(19) Europäisches Patentamt
European Patent Office
Office européen des brevets

(11) Veröffentlichungsnummer: **0 004 303**
**B1**

(12) # EUROPÄISCHE PATENTSCHRIFT

(45) Veröffentlichungstag der Patentschrift: **13.01.82**

(21) Anmeldenummer: **79100633.1**

(22) Anmeldetag: **05.03.79**

(51) Int. Cl.³: **C 07 D 233/60**, C 07 D 249/08, A 01 N 43/56, A 01 N 43/64

(54) **Acylierte 1-Azolyl-2-hydroxy-butan-Derivate, Verfahren zu ihrer Herstellung und ihre Verwendung als Fungizide.**

(30) Priorität: **18.03.78 DE 2811919**

(43) Veröffentlichungstag der Anmeldung:
**03.10.79 Patentblatt 79/20**

(45) Bekanntmachung des Hinweises auf die Patenterteilung:
**13.01.82 Patentblatt 82/2**

(84) Benannte Vertragsstaaten:
**BE CH DE FR GB IT NL SE**

(56) Entgegenhaltungen:
DE - A - 2 600 799
DE - A - 2 632 602
DE - A - 2 632 603
DE - A - 2 635 663
DE - A - 2 635 664
DE - A - 2 635 665
DE - A - 2 635 666

(73) Patentinhaber: **BAYER AG**
**Zentralbereich Patente, Marken und Lizenzen**
**D-5090 Leverkusen 1, Bayerwerk (DE)**

(72) Erfinder: **Krämer, Wolfgang, Dr.**
**Am Eckbusch 39/162**
**D-5600 Wuppertal 1 (DE)**
Erfinder: **Büchel, Karl Heinz, Prof. Dr.**
**Bergerheide 62**
**D-5600 Wuppertal 1 (DE)**
Erfinder: **Frohberger, Paul-Ernst, Dr.**
**Willi-Baumeister-Strasse 5**
**D-5090 Leverkusen 1 (DE)**
Erfinder: **Brandes, Wilhelm, Dr.**
**Eichendorffstrasse 3**
**D-5653 Leichlingen 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 004 303 B1

Courier Press, Leamington Spa, England.

## Acylierte 1-Azolyl-2-hydroxy-butan-Derivate, Verfahren zu ihrer Herstellung und ihre Verwendung als Fungizide

Die Erfindung betrifft neue acylierte 1-Azolyl-2-hydroxy-butan-Derivate, mehrere Verfahren zu ihrer Herstellung und ihre Verwendung als Fungizide.

Es ist bereits bekannt geworden, daß acylierte Triazolyl- und Imidazolyl-O,N-acetale, wie insbesondere im Phenylteil substituierte 2-Acyloxy-3,3-dimethyl-1-phenoxy-1-(1,2,4-triazol-1-yl)- bzw. -(imidazol-1-yl)-butane, gute fungizide Eigenschaften aufweisen (vgl. DE—OS 2 600 799 bzw. DE—OS 2 604 761. Deren Wirkung ist jedoch, insbesondere bei niedrigen Aufwandmengen und -konzentrationen, nicht immer ganz befriedigend.

Es wurden neue acylierte 1-Azolyl-2-hydroxy-butan-Derivate der allgemeinen Formel I

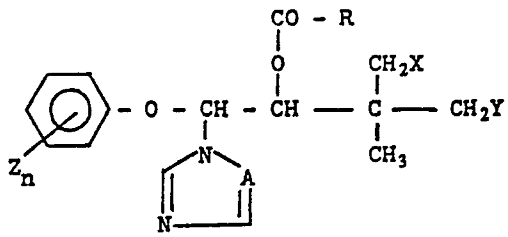

(I)

in welcher

A für ein Stickstoffatom oder die CH-Gruppe steht,

R für Alkyl mit 1 bis 6, Alkenyl und Alkinyl mit jeweils 2 bis 4 Kohlenstoffatomen, Halogenalkyl mit 1 bis 2 Kohlenstoff- und 1 bis 5 Halogenatomen, Alkoxy und Alkoxyalkyl mit 1 bis 4 Kohlenstoffatomen in den Alkylteilen steht, ferner für Phenyl und Phenylalkyl mit bis zu 2 Kohlenstoffatomen im Alkylteil steht, wobei die beiden letztgenannten Reste im Phenylteil substituiert sein können durch Halogen, Cyano, Nitro, Methyl und Ethyl, ferner für Alkylamino mit 1 bis 12 Kohlenstoffatomen und für Dimethyl- oder Diethylamino, Halogenalkylamino mit bis zu 4 Kohlenstoff- und 5 Halogenatomen steht, für Alkoxycarbonylamino und Alkoxyalkylamino mit jeweils 1 bis 4 Kohlenstoffatomen in jedem Alkylteil und endlich für Phenylamino steht, welches gegebenfalls durch Halogen, Nitro, Cyano, Alkyl mit bis zu 4, Alkoxy und Alkoxythio mit bis zu 2 Kohlenstoffatomen, Halogenalkyl mit bis zu 2 Kohlenstoff- und 5 Halogenatomen und durch Alkoxycarbonylalkenyl mit bis zu 4 Kohlenstoffatomen in den Alkyl- und Alkenylteilen substituiert sein kann;

X und Y für Halogen und Alkylcarbonyloxy mit 1 bis 4 Kohlenstoffatomen im Alkylteil stehen und darüber hinaus X noch für Wasserstoff stehen kann, für Halogen, Cyano, Nitro, Alkyl mit bis zu 4 Kohlenstoffatomen, für Cyclohexyl, für Halogenalkyl mit bis zu 2 Kohlenstoff- und 5 Halogenatomen, für Alkoxy mit bis zu 2 und Alkoxycarbonyl mit insgesamt bis zu 5 Kohlenstoffatomen steht, und weiterhin für Phenyl oder Phenoxy steht, wobei diese beiden Reste gegebenenfalls durch Halogen, Amino, Cyano, Nitro, Methyl und Ethyl substituiert sein können, und

n für Zahlen von 0 bis 3 steht,

und deren physiologisch verträglichen Säureadditions-Salze und Metallsalz-Komplexe gefunden. Sie weisen starke fungizide Eigenschaften auf.

Die Verbindung der allgemeinen Formel I besitzen zwei asymmetrische Kohlenstoffatome; sie können deshalb in der erythro- wie in der threo-Form vorliegen. In beiden Fällen liegen sie vorwiegend als Racemate vor.

Man erhält die acylierten 1-Azolyl-2-hydroxy-butan-Derivate der allgemeinen Formel I, wenn man 1-Azolyl-2-hydroxy-butan-Derivate der allgemeinen Formel II

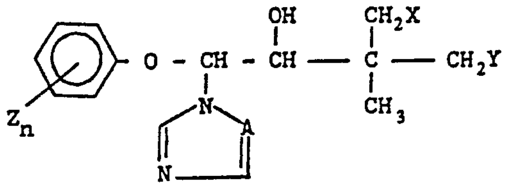

(II)

in welcher

A, X, Y, Z und n die oben angegebene Bedeutung haben,

(a) mit Säurehalogeniden der allgemeinen Formel III

Hal—CO—R (III)

in welcher

R die oben angegebene Bedeutung hat und

Hal für Halogen, insbesondere Chlor oder Brom steht,

gegebenenfalls in Gegenwart eines Lösungsmittels und gegebenenfalls in Gegenwart eines Säurebindemittels umsetzt, oder
(b) mit Säureanhydriden der allgemeinen Formel IV

R—CO—O—CO—R (IV)

in welcher
R die oben angegebene Bedeutung hat,
in Gegenwart eines Lösungsmittels und gegebenenfalls in Gegenwart eines Katalysators umsetzt, oder
(c) mit Ketenen der allgemeinen Formel V

$$O{=}C{=}C(R')(R'') \quad (V)$$

in welcher
R′ für Wasserstoff, für Alkyl mit bis zu 5 oder für Alkenyl oder Alkoxy mit bis zu 3 Kohlenstoffatomen steht, ferner für Phenyl steht, welches durch Halogen, Cyano, Nitro und Alkyl mit bis zu 2 Kohlenstoffatomen substituiert sein kann, sodann für Halogenmethyl mit 1 bis 3 Fluor- und/oder Chloratomen steht, und schließlich für Chlor oder Brom steht, und die unter R′ genannten Bedeutungen annehmen kann, jedoch mit der Maßgabe, daß die Summe der Kohlenstoffatome in den Alkyl-, Alkenyl- und Alkoxy-Resten für R′ und R″ gemeinsam um die Zahl 1 unter der Zahl derjenigen Kohlenstoffatome, die bei der R-Definition in Anspruch 1 für die genannten Reste angegeben ist, bleiben muß, und daß höchstens einer der Reste R′ und R″ für gegebenenfalls wie angegeben substituiertes Phenyl oder Halogenmethyl stehen darf,
in Gegenwart eines Lösungsmittels und gegebenenfalls in Gegenwart eines Katalysators umsetzt, oder
(d) mit Isocyanaten der allgemeinen Formel VI

O=C=N—R″ (VI)

in welcher
R‴ für Alkyl mit bis zu 6, für Halogenalkyl mit bis zu 2 Kohlenstoff- und bis zu 5 Halogenatomen steht, für Alkoxycarbonyl oder Alkoxyalkyl mit jeweils bis zu 4 Kohlenstoffatomen in jedem Alkylteil steht, oder für Phenyl steht, welches durch Halogen, Cyano, Nitro und Alkyl mit bis zu 4, Alkoxy und Alkylthio mit bis zu 2 Kohlenstoffatomen, Halogenalkyl mit bis zu 2 Kohlenstoff- und bis zu 5 Halogenatomen und/oder durch Alkoxycarbonylalkenyl mit bis zu 4 Kohlenstoffatomen im Alkyl- oder Alkenyl-Teil substituiert sein kann,
in Gegenwart eines Lösungsmittels und gegebenenfalls in Gegenwart eines Katalysators umsetzt.

Weiterhin können die erfindungsgemäß erhältlichen acylierten 1-Azolyl-2-hydroxy-butan-Derivate der allgemeinen Formel I durch Umsetzen mit Säuren in die Salze überführt werden, bzw. können durch Reaktion mit Metallsalzen die entsprechenden Metallsalz-Komplexe erhalten werden.

In manchen Fällen erweist es sich als vorteilhaft, einzelne Verbindungen, in denen X oder/und Y für Alkylcarbonyloxy stehen, ausgehend von solchen Verbindungen der allgemeinen Formel II, in denen dann X oder/und Y für die Hydroxygruppe stehen, gemäß Verfahren (a) oder (b) herzustellen (vgl. auch Herstellungsbeispiele).

Ueberraschenderweise zeigen die erfindungsgemäßen acylierten 1-Azolyl-2-hydroxy-butan-Derivate eine erheblich höhere fungizide Wirksamkeit, insbesondere gegen Rost- und Mehltauarten, als die aus dem Stand der Technik bekannten acylierten Triazolyl- und Imidazolyl-O,N-acetale, welche chemisch und wirkungsmäßig naheliegendste Verbindungen sind. Die erfindungsgemäßen Stoffe stellen somit eine Bereicherung der Technik dar.

Ganz besonders bevorzugt sind diejenigen Verbindungen der allgemeinen Formel I, in denen R für Methyl, Ethyl, Isobutyl, Chlormethyl, Dichlormethyl, Methacryl, gegebenenfalls einfach oder mehrfach substituiertes Phenyl mit Chlor, Brom oder Methyl als Substituenten, farner für Methoxy, Ethoxy, Isopropoxy, Butoxy oder Isobutoxy, Methyl- und Ethylamino, Dimethylamino, Phenylamino, Chlorphenylamino, Chlorethylamino, Methoxycarbonylamino, Ethoxycarbonylamino und Methoxymethylamino steht, X für Wasserstoff, Chlor, Brom oder Methylcarbonyloxy steht, Y für Chlor, Brom oder Methylcarbonyloxy steht, Z für Chlor, Brom, Methyl, Ethyl, Cyclohexyl, Methoxy, Methylthio, Trifluormethyl, Methoxycarbonyl, Cyano, Nitro sowie gegebenenfalls durch Chlor substituiertes Phenyl oder Phenoxy steht und n für 0, 1 oder 2 steht.

Im Einzelnen seien außer den Herstellungsbeispielen und den Beispielen der Tabelle 1 folgende Verbindungen der allgemeinen Formel I genannt:
1-(4-Bromphenoxy)-1-(1,2,4-triazol-1-yl)-2-isobutylcarbonyloxy-3,3-dimethyl-4-chlor-butan
1-(2,4-Dichlorphenoxy)-1-(1,2,4-triazol-1-yl)-2-methyl-carbamoyloxy-3,3-dimethyl-4-chlor-butan
1-(2,4-Dichlorphenoxy)-1-(1,2,4-triazol-1-yl)-2-äthyl-carbonyloxy-3,3-dimethyl-4-chlor-butan
1-(4-Methoxyphenoxy)-1-(1,2,4-triazol-1-yl)-2-acetoxy-3,3-dimethyl-4-chlor-butan

1-(3-Trifluormethylphenoxy)-1-(1,2,4-triazol-1-yl)-2-chloracetoxy-3,3-dimethyl-4-chlor-butan
1-(4-Methoxycarbonylphenoxy)-1-(1,2,4-triazol-1-yl)-2-dichloracetoxy-3,3-dimethyl-4-brom-butan
1-(4-Biphenylyloxy)-1-(1,2,4-triazol-1-yl)-2-methylcarbamoyloxy-3,3-dimethyl-4-chlor-butan
1-(4-Bromphenoxy)-1-(1,2,4-triazol-1-yl)-2-isobutylcarbonyloxy-3,3-dimethyl-4-brom-butan
1-(2,4-Dichlorphenoxy)-1-(1,2,4-triazol-1-yl)-2-methylcarbamoyloxy-3,3-dimethyl-4-brom-butan
1-(2,4-Dichlorphenoxy)-1-(1,2,4-triazol-1-yl)-2-äthylcarbonyloxy-3,3-dimethyl-4-brom-butan
1-(4-Methoxyphenoxy)-1-(1,2,4-triazol-1-yl)-2-acetoxy-3,3-dimethyl-4-brom-butan
1-(3-Trifluormethylphenoxy)-1-(1,2,4-triazol-1-yl)-2-chloracetoxy-3,3-dimethyl-4-brom-butan
1-(4-Methoxycarbonylphenoxy)-1-(1,2,4-triazol-1-yl)-2-dichloracetoxy-3,3-dimethyl-4-chlor-butan
1-(4-Biphenylyloxy)-1-(1,2,4-triazol-1-yl)-2-methylcarbamoyloxy-3,3-dimethyl-4-brom-butan
1-(4-Biphenylyloxy)-1-(1,2,4-triazol-1-yl)-2-methylcarbamoyloxy-3,3-dimethyl-4-acetoxy-butan
1-(4-Biphenylyloxy)-1-(1,2,4-triazol-1-yl)-2-äthylcarbamoyloxy-3,3-dimethyl-4-chlor-butan
1-(4-Biphenylyloxy)-1-(1,2,4-triazol-1-yl)-2-äthylcarbamoyloxy-3,3-dimethyl-4-brom-butan
1-(4-Biphenylyloxy)-1-(1,2,4-triazol-1-yl)-2-äthylcarbamoyloxy-3,3-dimethyl-4-acetoxy-butan
1-(4-Biphenylyloxy)-1-(1,2,4-triazol-1-yl)-2-methoxymethylcarbamoyloxy-3,3-dimethyl-4-chlor-butan
1-(4-Biphenylyloxy)-1-(1,2,4-triazol-1-yl)-2-methoxymethylcarbamoyloxy-3,3-dimethyl-4-brom-butan
1-(4-Biphenylyloxy)-1-(1,2,4-triazol-1-yl)-2-methoxymethylcarbamoyloxy-3,3-dimethyl-4-acetoxy-butan
1-(4-Biphenylyloxy)-1-(1,2,4-triazol-1-yl)-2-äthoxymethylcarbamoyloxy-3,3-dimethyl-4-chlor-butan
1-(4-Biphenylyloxy)-1-(1,2,4-triazol-1-yl)-2-äthoxymethylcarbamoyloxy-3,3-dimethyl-4-brom-butan
1-(4-Biphenylyloxy)-1-(1,2,4-triazol-1-yl)-2-äthoxymethylcarbamoyloxy-3,3-dimethyl-4-acetoxy-butan
1-(4-Biphenylyloxy)-1-(1,2,4-triazol-1-yl)-2-methoxycarbonylcarbamoyloxy-3,3-dimethyl-4-chlor-butan
1-(4-Biphenylyloxy)-1-(1,2,4-triazol-1-yl)-2-methoxycarbonylcarbamoyloxy-3,3-dimethyl-4-brom-butan
1-(4-Biphenylyloxy)-1-(1,2,4-triazol-1-yl)-2-äthoxycarbonylcarbamoyloxy-3,3-dimethyl-4-chlor-butan
1-(4-Biphenylyloxy)-1-(1,2,4-triazol-1-yl)-2-äthoxycarbonylcarbamoyloxy-3,3-dimethyl-4-brom-butan
1-(4-Biphenylyloxy)-1-(1,2,4-triazol-1-yl)-2-phenylcarbamoyloxy-3,3-dimethyl-4-chlor-butan
1-(4-Biphenylyloxy)-1-(1,2,4-triazol-1-yl)-2-phenylcarbamoyloxy-3,3-dimethyl-4-brom-butan
1-(4-Biphenylyloxy)-1-(1,2,4-triazol-1-yl)-2-(4-chlorphenylcarbamoyloxy)-3,3-dimethyl-4-chlor-butan
1-(4-Biphenylyloxy)-1-(1,2,4-triazol-1-yl)-2-(4-chlorphenylcarbamoyloxy)-3,3-dimethyl-4-brom-butan
1-(4-Biphenylyloxy)-1-(1,2,4-triazol-1-yl)-2-dimethylcarbamoyloxy-3,3-dimethyl-4-chlor-butan
1-(4-Biphenylyloxy)-1-(1,2,4-triazol-1-yl)-2-dimethylcarbamoyloxy-3,3-dimethyl-4-brom-butan
1-(4-Biphenylyloxy)-1-(1,2,4-triazol-1-yl)-2-dimethylcarbamoyloxy-3,3-dimethyl-4-acetoxy-butan
1-(4-Biphenylyloxy)-1-(1,2,4-triazol-1-yl)-2-methoxycarbonyloxy-3,3-dimethyl-4-chlor-butan
1-(4-Biphenylyloxy)-1-(1,2,4-triazol-1-yl)-2-methoxycarbonyloxy-3,3-dimethyl-4-brom-butan
1-(4′-Chlor-4-biphenylyloxy)-1-(1,2,4-triazol-1-yl)-2-acetoxy-3,3-dimethyl-4-chlor-butan
1-(4′-Chlor-4-biphenylyloxy)-1-(1,2,4-triazol-1-yl)-2-acetoxy-3,3-dimethyl-4-brom-butan
1-(4′-Chlor-4-biphenylyloxy)-1-(1,2,4-triazol-1-yl)-2,4-diacetoxy-3,3-dimethyl-butan
1-(4-Phenoxyphenoxy)-1-(1,2,4-triazol-1-yl)-2-acetoxy-3,3-dimethyl-4-chlor-butan
1-(4′-Chlor-4-phenoxyphenoxy)-1-(1,2,4-triazol-1-yl)-2-acetoxy-3,3-dimethyl-4-chlor-butan
1-(4-Cyanophenoxy)-1-(1,2,4-triazol-1-yl)-2-acetoxy-3,3-dimethyl-4-chlor-butan
1-(4-Nitrophenoxy)-1-(1,2,4-triazol-1-yl)-2-acetoxy-3,3-dimethyl-4-chlor-butan
1-(4′-Chlor-4-biphenylyloxy)-1-(1,2,4-triazol-1-yl)-2-acetoxy-3,3-di(chlormethyl)-butan
1-(4-Biphenylyloxy)-1-(1,2,4-triazol-1-yl)-2-methylcarbamoyloxy-3,3-di(chlormethyl)-butan
1-(4-Biphenylyloxy)-1-(1,2,4-triazol-1-yl)-2-methylcarbamoyloxy-3,3-dimethyl-4-brom-butan
1-(4-Chlorphenoxy)-1-(1,2,4-triazol-1-yl)-2-methacrylcarbonyloxy-3,3-dimethyl-4-brom-butan
1-(4-Chlorphenoxy)-1-(1,2,4-triazol-1-yl)-2,4-diacetoxy-3,3-dimethyl-butan
1-(4-Chlorphenoxyl)-1-(1,2,4-triazol-1-yl)-2,4-diacetoxy-3-acetoxymethyl-3-methyl-butan
1-(4-Bromphenoxy)-1-(imidazol-1-yl)-2-isobutylcarbonyloxy-3,3-dimethyl-4-chlor-butan
1-(2,4-Dichlorphenoxy)-1-(imidazol-1-yl)-2-methylcarbamoyloxy-3,3-dimethyl-4-chlor-butan
1-(2,4-Dichlorphenoxy)-1-(imidazol-1-yl)-2-äthylcarbonyloxy-3,3-dimethyl-4-chlor-butan
1-(4-Methoxyphenoxy)-1-(imidazol-1-yl)-2-acetoxy-3,3-dimethyl-4-chlor-butan
1-(3-Trifluormethylphenoxy)-1-(imidazol-1-yl)-2-chloracetoxy-3,3-dimethyl-4-chlor-butan
1-(4-Methoxycarbonylphenoxy)-1-(imidazol-1-yl)-2-dichloracetoxy-3,3-dimethyl-4-brom-butan
1-(4-Biphenylyloxy)-1-(imidazol-1-yl)-2-methylcarbamoyloxy-3,3-dimethyl-4-chlor-butan
1-(4-Biphenylyloxy)-1-(imidazol-1-yl)-2-methylcarbamoyloxy-3,3-dimethyl-4-brom-butan
1-(4-Biphenylyloxy)-1-(imidazol-1-yl)-2-methylcarbamoyloxy-3,3-dimethyl-4-acetoxy-butan
1-(4-Biphenylyloxy)-1-(imidazol-1-yl)-2-äthylcarbamoyloxy-3,3-dimethyl-4-chlor-butan
1-(4-Biphenylyloxy)-1-(imidazol-1-yl)-2-äthylcarbamoyloxy-3,3-dimethyl-4-brom-butan
1-(4-Biphenylyloxy)-1-(imidazol-1-yl)-2-äthylcarbamoyloxy-3,3-dimethyl-4-acetoxy-butan
1-(4-Biphenylyloxy)-1-(imidazol-1-yl)-2-methoxymethylcarbamoyloxy-3,3-dimethyl-4-chlor-butan
1-(4-Biphenylyloxy)-1-(imidazol-1-yl)-2-methoxymethylcarbamoyloxy-3,3-dimethyl-4-brom-butan
1-(4-Biphenylyloxy)-1-(imidazol-1-yl)-2-methoxymethylcarbamoyloxy-3,3-dimethyl-4-acetoxy-butan
1-(4-Biphenylyloxy)-1-(imidazol-1-yl)-2-äthoxymethylcarbamoyloxy-3,3-dimethyl-4-chlor-butan

1-(4-Biphenylyloxy)-1-(imidazol-1-yl)-2-äthoxymethylcarbamoyl-3,3-dimethyl-4-brom-butan
1-(4-Biphenylyloxy)-1-(imidazol-1-yl)-2-äthoxymethylcarbamoyloxy-3,3-dimethyl-4-acetoxy-butan
1-(4-Biphenylyloxy)-1-(imidazol-1-yl)-2-methoxycarbonylcarbamoyloxy-3,3-dimethyl-4-chlor-butan
1-(4-Biphenylyloxy)-1-(imidazol-1-yl)-2-methoxycarbonylcarbamoyloxy-3,3-dimethyl-4-brom-butan
1-(4-Biphenylyloxy)-1-(imidazol-1-yl)-2-äthoxycarbonylcarbamoyloxy-3,3-dimethyl-4-chlor-butan
1-(4-Biphenylyloxy)-1-(imidazol-1-yl)-2-äthoxycarbonylcarbamoyloxy-3,3-dimethyl-4-brom-butan
1-(4-Biphenylyloxy)-1-(imidazol-1-yl)-2-phenylcarbamoyloxy-3,3-dimethyl-4-chlor-butan
1-(4-Biphenylyloxy)-1-(imidazol-1-yl)-2-phenylcarbamoyloxy-3,3-dimethyl-4-brom-butan
1-(4-Biphenylyloxy)-1-(imidazol-1-yl)-2-(4-chlor-phenylcarbamoyloxy-3,3-dimethyl-4-chlor-butan
1-(4-Biphenylyloxy)-1-(imidazol-1-yl)-2-(4-chlorphenylcarbamoyloxy)-3,3-dimethyl-4-brom-butan
1-(4-Biphenylyloxy)-1-(imidazol-1-yl)-2-dimethylcarbamoyloxy-3,3-dimethyl-4-chlor-butan
1-(4-Biphenylyloxy)-1-(imidazol-1-yl)-2-dimethylcarbamoyloxy-3,3-dimethyl-4-brom-butan
1-(4-Biphenylyloxy)-1-(imidazol-1-yl)-2-dimethylcarbamoyloxy-3,3-dimethyl-4-acetoxy-butan
1-(4-Biphenylyloxy)-1-(imidazol-1-yl)-2-methoxycarbonyloxy-3,3-dimethyl-4-chlor-butan
1-(4-Biphenylyloxy)-1-(imidazol-1-yl)-2-methoxycarbonyloxy-3,3-dimethyl-4-brom-butan

Verwendet man beispielsweise 1-(4-Chlorphenoxy)-1-(1,2,4-triazol-1-yl)-3,3-dimethyl-4-chlor-butan-2-ol und Dichloracetylchlorid als Ausgangsstoffe, so kann der Reaktionsablauf durch das folgende Formelschema wiedergegeben werden

(Verfahren a):

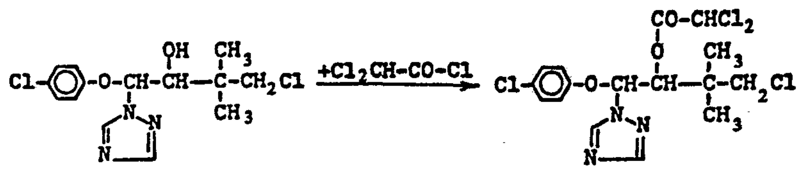


Verwendet man beispielsweise 1-(4-Chlorphenoxyl)-1-(imidazol-1-yl)-3,3-dimethyl-4-chlor-butan-2-ol und Acetanhydrid als Ausgangsstoffe, so kann der Reaktionsablauf durch das folgende Formelschema wiedergegeben werden

(Verfahren b):

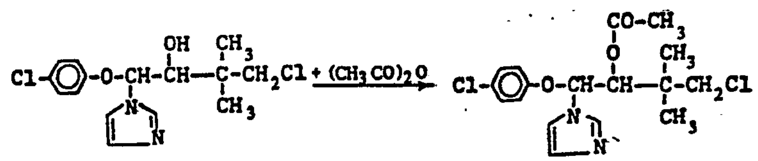


Verwendet man beispielsweise 1-(2,4-Dichlorphenoxy)-1-(1,2,4-triazol-1-yl)-3,3-dimethyl-4-brom-butan-2-ol und 4-Chlorphenylisocyanat als Ausgangsstoffe, so kann der Reaktionsablauf durch das folgende Formelschema wiedergegeben werden

(Verfahren d):

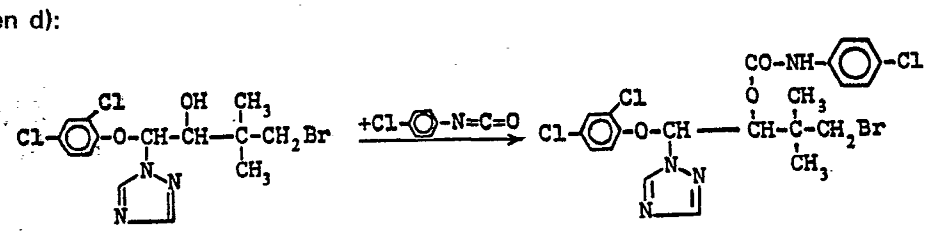

Umsetzungen von 1-Azolyl-2-hydroxy-butan-Derivaten der allgemeinen Formel II mit einem Keten der allgemeinen Formel V lassen sich in entsprechender Weise formulieren (Verfahren c).

Die für alle Verfahrensvarianten als Ausgangsstoffe zu verwendenden 1-Azolyl-2-hydroxy-butan-Derivate sind durch die allgemeine Formel II definiert.

Die erfindungsgemäß verwendbaren 1-Azolyl-2-hydroxy-butan-Derivate der allgemeinen Formel II sind bekannt (vgl. DE—OS 26 32 603, DE—OS 26 32 602 und DE—OS 26 35 666), bzw. können nach den dort beschriebenen Verfahren erhalten werden, indem man die entsprechenden 1-Brom-butan-2-one zunächst mit 1,2,4-Triazol oder Imidazol in Gegenwart eines Verdünnungsmittels, wie beispielsweise Aceton, und in Gegenwart eines Säurebindemittels, wie beispielsweise Kaliumcarbonat, bei Temperaturen zwischen 60 und 120°C umsetzt, und die dabei erhaltenen 1-Azolyl-butan-2-one in an sich bekannter Weise mit komplexen Hydriden, wie beispielsweise Natriumborhydrid, oder mit Aluminiumisopropylat, gegebenenfalls in Gegenwart eines Verdünnungsmittels, wie beispielsweise Aethanol, bei Temperaturen zwischen 0 und 30°C reduziert und in üblicher Weise isoliert.

Die für die Herstellung der erfindungsgemäßen Stoffe gemäß Verfahrensvariante (a) erforderlichen Säurehalogenide sind durch die allgemeine Formel III definiert.

Die Säurehalogenide der allgemeinen Formel III sind bekannt oder lassen sich nach üblichen Verfahren herstellen; so z.B. durch Umsetzung von Carbonsäuren bzw. deren Alkalisalzen mit Säurehalogeniden des Phosphors oder Schwefels. Diese Methoden sind aus den allgemeinen Lehrbüchern der organischen Chemie bekannt.

Die weiterhin für die Herstellung der erfindungsgemäßen Stoffe gemäß Verfahrenvariante (b) erforderlichen Säureanhydride sind durch die allgemeine Formel IV definiert.

Die Säureanhydride der allgemeinen Formel IV sind bekannt oder lassen sich nach bekannten Verfahren herstellen; so z.B. durch Einwirkung von Säurechloriden auf die Alkalisalze der Carbonsäuren. Diese Verfahren sind allgemein bekannt.

Die außerdem für die Herstellung der erfindungsgemäßen Stoffe gemäß Verfahrensvariante (c) erforderlichen Ketene sind durch die allgemeine Formel V definiert.

Die Ketene der allgemeinen Formel V sind bekannt oder lassen sich nach bekannten Verfahren herstellen, so z.B. durch Thermolyse von Ketonen oder durch Dehydratisierung von Carbonsäuren (vgl. Houben-Weyl, 'Methoden der organischen Chemie', band 7/4, Georg Thieme Verlag).

Die weiterhin für die Herstellung der erfindungsgemäßen Stoffe gemäß Verfahrensvariante (d) erforderlichen Isocyanate sind durch die allgemeine Formel VI definiert.

Die Isocyanate der allgemeinen Formel VI sind bekannt oder lassen sich nach allgemein üblichen und bekannten Verfahren herstellen; so z.B. durch Umsetzen von Aminen mit Phosgen und anschließendem Erhitzen.

Als Lösungsmittel kommen für die Umsetzung gemäß Verfahrensvariante (a) vorzugsweise alle inerten organischen Lösungsmittel infrage. Hierzu gehören vorzugsweise Ketone, wie Diäthylketon, insbesonder Aceton und Methyläthylketon; Nitrile, wie Propionitril, insbesondere Acetonitril; Aether, wie Tetrahydrofuran oder Dioxan; Ester, wie Essigsäureäthylester; aromatische Kohlenwasserstoffe, wie Benzol oder Toluol und halogenierte Kohlenwasserstoffe, wie Methylenchlorid, Tetrachlorkohlenstoff oder Chloroform. Der Einfachheit halber kann das eingesetzte Säurechlorid auch als Lösungmittel verwendet werden, wodurch ein entsprechender Ueberschuß notwendig wird.

Die Reaktionstemperaturen können bei der Durchführung der Verfahrensvariante (a) in einem größeren Bereich variiert werden. Im allgemeinen arbeitet man zwischen 0 und 100°C, vorzugsweise zwischen 20 und 85°C. Bei der Anwesenheit eines Lösungsmittels wird zweckmäßigerweise beim Siedepunkt des jeweiligen Lösungsmittels gearbeitet.

Das erfindungsgemäße Verfahren (a) kann gegebenenfalls in Gegenwart von Säurebindem (Halogenwasserstoff-Akzeptoren) durchgeführt werden; als solche können alle üblichen Säurebindungsmittel verwendet werden. Hierzu gehören organische Basen, vorzugsweise tertiäre Amine, wie z.B. Triäthylamin; ferner anorganische Basen, wie z.B. Alkalihydroxide und Alkalicarbonate.

Bei der Durchführung der Verfahrensvariante (a) arbeitet man vorzugsweise in molaren Mengen. Die Verbindungen der allgemeinen Formel I fallen in Form ihrer Hydrohalogenide an und können als solche isoliert werden, indem man sie durch Zugabe eines organischen Solvents, z.B. Hexan ausfällt, absaugt und gegebenenfalls durch Umkristallisation reinigt. Die Verbindungen der allgemeinen Formel I können auch in Form ihrer freien Base isoliert werden, indem man das Reaktionsgemisch mit wässriger Natriumhydrogencarbonatlösung versetzt und die Base nach üblichen Methoden isoliert.

Als Verdünnungsmittel kommen für die Umsetzung gemäß Verfahrensvariante (b) vorzugsweise alle inerten organischen Lösungsmittel infrage. Hierzu gehören vorzugsweise die bei der Verfahrensvariante (a) aufgezählten Solventien sowie die jeweils verwendeten Säureanhydride der allgemeinen Formel IV.

Als Katalysatoren können bei den Verfahrensvarianten (b) und (c) vorzugsweise alle üblichen sauren und basischen Katalysatoren verwendet werden, wie z.B. Schwefelsäure, Chlorwasserstoff, Bromwasserstoff, Bortrifluorid, Zinkchlorid, Natriumacetat, Natriumbenzoat, Natriumcarbonat, Calciumoxid, Magnesiumoxid.

Die Reaktionstemperaturen können bei der Durchführung der Verfahrensvariante (b) in einem größeren Bereich variiert werden. Im allgemeinen arbeitet man zwischen 0 und 150°C, vorzugsweise zwischen 80 und 120°C.

Bei der Durchführung der Verfahrensvariante (b) arbeitet man vorzugsweise in molaren Mengen. Der Einfachheit halber kann man das eingesetzte Säureanhydrid der allgemeinen Formel IV auch als Lösungsmittel verwenden, womit ein entsprechender Ueberschuß erforderlich wird. Die Isolierung der Verbindungen der allgemeinen Formel I erfolgt in üblicher Weise.

Als Verdünnungsmittel kommen für die Umsetzung gemäß Verfahrensvariante (c) vorzugsweise alle inerten organischen Lösungsmittel infrage. Hierzu gehören vorzugsweise die bei der Verfahrensvariante (a) aufgeführten Solventien.

Die Reaktionstemperaturen können bei der Durchführung der Verfahrensvariante (c) in einem bestimmten Bereich variiert werden. Im allgemeinen arbeitet man zwischen −10 und 70°C, vorzugsweise zwischen 0 und 40°C.

Als Verdünnungsmittel kommen für die Umsetzung gemäß Verfahrensvariante (d) vorzugsweise alle inerten organischen Lösungsmittel infrage. Hierzu gehören vorzugsweise die bei der Verfahrensvariante (a) aufgezählten Solventien.

Als Katalysatoren können beim Verfahren (d) vorzugsweise verwendet werden: tertiäre Basen, wie Triäthylamin und Pyridin oder Zinn-organische Verbindungen, wie Dibutylzinndilaurat.

Die Reaktionstemperaturen können bei der Durchführung der Verfahrensvariante (d) in einem größeren Bereich variiert werden. Im allgemeinen arbeitet man zwischen 0 und 100°C, vorzugsweise zwischen 20 und 40°C.

Bei der Durchführung der Verfahrensvariante (d) arbeitet man vorzugsweise in molaren Mengen. Zur Isolierung der Verbindungen der allgemeinen Formel I wird das Lösungsmittel abdestilliert und der Rückstand nach üblichen Methoden aufgearbeitet.

Zur Herstellung von Säureadditionssalzen der Verbindungen der allgemeinen Formel I kommen alle physiologisch verträglichen Säuren infrage. Hierzu gehören vorzugsweise die Halogenwasserstoffsäuren, wie z.B die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure, Milchsäure, sowie Sulfonsäuren, wie z.B. p-Toluolsulfonsäure und 1,5-Naphthalindisulfonsäure.

Die Salze der Verbindungen der allgemeinen Formel I können in einfacher Weise nach üblichen Salzbildungsmethoden, z.B. durch Lösen einer Verbindung der allgemeinen Formel I in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der allgemeinen Formel I kommen vorzugsweise Salze von Metallen der II. bis IV.-Haupt- und der I. und II. sowie VI. bis VIII. Nebengruppe infrage, wobei Kupfer, Zink, Mangen, Magnesium, Zinn, Eisen und Nickel beispielhaft genannt seien. Als Anionen der Salze kommen solche in Betracht, die sich von physiologischen Säuren ableiten. Hierzu gehören vorzugsweise die Halogenwasserstoffsäuren, wie z.B. die Chlorwasserstoffsäure und die Bromwasserstoffsäure, ferner Phosphorsäure, Salpetersäure und Schwefelsäure.

Die Metallsalzkomplexe der Verbindungen der allgemeinen Formel I können in einfacher Weise nach üblichen Verfahren erhalten werden, so z.B. durch Lösen Metallsalzes in Alkohol, z.B. Aethanol, und Hinzufügen zur Verbindung der allgemeinen Formel I. Man kann Metallsalzkomplexe in bekannter Weise, z.B. durch Abfiltrieren, isolieren und gegebenenfalls durch Umkristallisieren reinigen.

Die erfindungsgemäßen Wirkstoffe weisen eine starke fungitoxische Wirkung auf. Sie schädigen Kulturpflanzen in den zur Bekämpfung von Pilzen notwendigen Konzentrationen nicht. Aus diesem Grunde sind sie für den Gebrauch als Pflanzenschutzmittel zur Bekämpfung von Pilzen geeignet. Fungitoxische Mittel im Pflanzenschutz werden eingesetzt zur Bekämpfung von Plasmodiophoromycetes, Chytridiomycetes, Zygomycetes, Oomycetes, Ascomycetes, Basidiomycetes, Deuteromycetes.

Die erfindungsgemäßen Wirkstoffe haben ein breites Wirkungsspektrum und können angewandt werden gegen parasitäre Pilze, die oberirdische Pflanzenteile befallen und die Pflanzen vom Boden her angreifen, sowie gegen samenübertagbare Krankheitserreger. Eine besonders gute Wirksamkeit entfalten sie gegen parasitäre Pilze auf oberirdischen Pflanzenteilen.

Als Pflanzenschutzmittel können die erfindungsgemäßen Wirkstoffe mit besonders gutem Erfolg zur Bekämpfung solcher Pilze eingesetzt werden, die echte Mehltauerkrankungen hervorrufen; so zur Bekämpfung von Podosphaera-Arten, wie z.B. gegen den Erreger des Apfelmehltaus (Podosphaera leucctricha), von Erysiphe-Arten, wie z.B. gegen den Erreger des Gurkenmehltaus (Erysiphe cichoracearum) oder des Gerstenmehltaus bzw. des Getreidemehltaus (Erysiphe graminis); sowie zur Bekämpfung anderer Getreidekrankheiten, wie Getreiderost. Besonders hervorzuheben ist, daß die erfindungsgemäßen Wirkstoffe nicht nur eine protektive Wirkung entfalten, sondern teilweise auch systemisch wirksam sind. So gelingt es, Pflanzen gegen Pilzbefall zu schützen, wenn man den Wirkstoff über den Boden und die Wurzel oder über das Saatgut den oberirdischen Teilen der Pflanze zuführt.

Als Pflanzenschutzmittel können die erfindungsgemäßen Wirkstoffe zur Saatgut- oder Bodenbehandlung und zur Behandlung oberirdischer Pflanzenteile benutzt werden.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen, und/oder festen Trägerstoffen, gegebenenfalls unter Verwendung von oblerflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen in wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wies Cyclohexan oder Paraffine, z.B. Erdölfraktionen, Alkohole, wie Butanol oder Glycol sowie deren Äther und Ester,

Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quartz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit, sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage; z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweßhydrolysate; als Dispergiermittel kommen in Frage; z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertigen Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschäumen, Verstreichen, Stäuben, Streuen, Trockenbeizen, Feuchtbeizen, Naßbeizen, Schlämmbeizen oder Inkrustieren.

Bei der Verwendung als Blattfungizide können die Wirkstoffkonzentrationen in den Anwendungsformen in einem größeren Bereich veriiert werden. Sie liegen im allgemeinen zwischen 0,1 und 0,00001 Gewichtsprozent. Vorzugsweise zwischen 0,05 und 0,0001%.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffmengen von 0,00001 bis 0,1 Gewichtsprozent, vorzugsweise 0,0001 bis 0,02% am Wirkungsort erforderlich.

Die vielseitigen Verwendungsmöglichkeiten gehen aus den nachfolgenden Beispielen hervor.

Beispiel A

Sproßbehandlungs-Test/Getreidemehltau/protektiv (blattzerstörende Mykose)

Zur Herstellung einer zweckmäßigen Wirkstoffzubereitung nimmt man 0,25 Gewichtsteile Wirkstoff in 25 Gewichsteilen Dimethylformamid und 0,06 Gewichtsteilen Alkylaryl-polyglykoläther auf und gibt 975 Gewichtsteile Wasser hinzu. Das Konzentrat verdünnt man mit Wasser auf die gewünschte Endkonzentration der Spritzbrühe.

Zur Prüfung auf protektive Wirksamkeit besprüht man die einblättrigen Gerstenjungpflanzen der Sorte Amsel mit der Wirkstoffzubereitung taufeucht. Nach Antrocknen bestäubt man die Gerstenpflanzen mit Sporen von Erysiphe graminis var.hordei.

Nach 6 Tagen Verweilzeit der Pflanzen bei einer Temperatur von 21—22°C und einer Luftfeuchtigkeit von 80—90% wertet man den Besatz der Pflanzen mit Mehltaupusteln aus. Der Befallsgrad wird in Prozenten des Befalls der unbehandelten Kontrollpflanzen ausgedrückt. Dabei bedeutet 0% keinen Befall und 100% den gleichen Befallsgrad bei der unbehandelten Kontrolle. Der Wirkstoff ist umso wirksamer, je geringer der Mehltaubefall ist.

Bei diesem Test zeigen z.B. folgende Verbindungen eine sehr gute Wirkung, die derjenigen der aus dem Stand der Technik bekannten Verbindungen überlegen ist: Verbindungen gemäß Herstellungsbeispielen: 4,3,5 und 6.

Beispiel B

Gerstenmehltau-Test (Erysiphe graminis var. hordei)/systemisch (pilzliche Getreidesproßkrankheit)

Die Anwendung der Wirkstoffe erfolgt als pulverförmige Saatgutbehandlungsmittel. Sie werden hergestellt durch Abstrecken das jeweiligen Wirkstoffes mit einem Gemisch aus gleichen Gewichsteilen Talkum und Kieselgur zu einer feinpulverigen Mischung mit der gewünschten Wirkstoffkonzentration.

Zur Saatgutbehandlung schüttelt man Gerstensaatgut mit dem abgestreckten Wirkstoff in einer verschlossenen Glasflasche. Das Saatgut sät man mit 3 x 12 Korn in Blumentöpfe 2 cm tief in ein Gemisch aus einem Volumteil Fruhstorfer Einheitserde und einem Volumteil Quarzsand ein. Die Keimung und der Auflauf erfolgen unter günstigen Bedingungen im Gewächshaus. 7 Tage nach der Aussaat, wenn die Gerstenpflanzen ihr erstes Blatt entfaltet haben, werden sie mit frischen Sporen von Erysiphe graminis var. hordei bestäubt und bei 21—22°C und 80—90% rel. Luftfeuchte und 16-stündiger Belichtung weiter kultiviert. Innerhalb von 6 Tagen bilden sich an den Blättern die typischen Mehltaupusteln aus.

Der Befallsgrad wird in Prozenten des Befalls der unbehandelten Kontrollpflanzen ausgedrückt. So bedeutet 0% keinen Befall und 100% den gleichen Befallsgrad wie bei der unbehandelten Kontrolle. Der Wirkstoff ist um so wirksamer je geringer der Mehltaubefall ist.

Wirkstoffe, Wirkstoffkonzentrationen im Saatgutbehandlungsmittel sowie dessen Aufwandmenge und der prozentuale Mehltaubefall werden ermittelt. Bei diesem Test zeigen z.B. folgende Verbindungen eine sehr gute Wirkung, die derjenigen der aus dem Stand der Technik bekannten Verbindungen überlegen ist:

Verbindungen gemäß Herstellungsbeispielen: 4, 3, 5, 6, 7, 8, 9 und 10.

Beispiel C

Sproßbehandlungs-Test/Getreiderost/protektiv (blattzerstörende Mykose)

Zur Herstellung einer zweckmäßigen Wirkstoffzubereitung nimmt man 0,25 Gewichtsteile Wirkstoff in 25 Gewichtsteilen Dimethylformamid und 0,06 Gewichtsteilen Alkyl-aryl-polyglykoläther auf und gibt 975 Gewichtsteile Wasser hinzu. Das Konzentrat verdünnt man mit Wasser auf die gewünschte Endkonzentration der Spritzbrühe.

Zur Prüfung auf protektive Wirksamkeit inokuliert man einblättrige Weizenjungpflanzen der Sorte Michigan Amber mit einer Uredosporensuspension von Puccinia recondita in 0,1% igem Wasseragar. Nach Antrocknen der Sporensuspension besprüht man die Weizenpflanzen mit der Wirkstoffzubereitung taufeucht und stellt sie zur Inkubation für 24 Stunden bei etwa 20°C und einer 100% igen Luftfeuchtigkeit in ein Gewächshaus.

Nach 10 Tagen Verweilzeit der Planzen bei einer Temperatur von 20°C und einer Luftfeuchtigkeit von 80—90% wertet man den Besatz der Pflanzen mit Rostpusteln aus. Der Befallsgrad wird in Prozenten des Befalls der unbehandelten Kontrollpflanzen ausgedrückt. Dabei bedeutet 0% keinen Befall und 100% den gleichen Befallsgrad wie bei der unbehandelten Kontrolle. Der Wirkstoff ist umso wirksamer, je geringer der Rostbefall ist.

Wirkstoffe, Wirkstoffkonzentrationen in der Spritzbrühe und Befallsgrade werden ermittelt. In diesem Test zeigen z.B. folgende Verbindungen eine sehr gute Wirkung, die derjenigen der aus dem Stand der Technik bekannten Verbindungen deutlich überlegen ist:

Verbindungen gemaß Herstellungsbeispielen: 4, 3, 6, 7, 9, 1 und 10.

Beispiel D

Erysiphe-Test (Gurken)/Protektiv

| | |
|---|---|
| Lösungsmittel: | 4,7 Gewichtsteile Aceton |
| Emulgator: | 0,3 Gewichtsteile Alkyl-arylpolyglykoläther |
| Wasser: | 95,0 Gewichtsteile |

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Gurkenpflanzen mit etwa drei Laubblättern bis zur Tropfnässe. Die Gurkenpflanzen verbleiben zur Trocknung 24 Stunden im Gewächshaus. Dann werden sie zur Inokulation mit Konidien des Pilzes Erysiphe cichoracearum bestäubt. Die Pflanzen werden anschliessend bei 23 bis 24°C einer relativen Luftfeuchtigkeit von ca. 75% im Gewächshaus aufgestellt.

Nach 12 Tagen wird der Befall der Gurkenpflanzen bestimmt. Die erhaltenen Boniturwerte werden in Prozent Befall umgerechnet. 0% bedeutet keinen Befall, 100% bedeutet, daß die Pflanzen vollständig befallen sind.

Wirkstoffe, Wirkstoffkonzentrationen und Ergebnisse werden ermittelt. Bei diesem Test zeigen z.B. folgende Verbindungen eine sehr gute Wirkung, die derjenigen der aus dem Stand der Technik bekannten Verbindungen überlegen ist:

Verbindungen gemäß Herstellungsbeispielen: 4, 3, 2, 5, 6, 7, 8, 9, 1 and 10.

Beispiel E
Podosphaera-Test (Apfel)/Protektiv

| | |
|---|---|
| Lösungsmittel: | 4,7 Gewichtsteile Aceton |
| Emulgator: | 0,3 Gewichtsteile Alkyl-arylpolyglykoläther |
| Wasser: | 95 Gewichtsteile |

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Apfelsämlinge, die sich im 4- bis 6-Blattstadium befinden, biz zur Tropfnässe. Die Pflanzen verbleiben 24 Stunden bei 20°C und einer relativen Luftfeuchtigkeit von 70% im Gewächshaus. Anschließend werden sie durch Bestäuben mit Konidien des Apfelmehltauerregers (Podosphaera leucotricha) inokuliert und in ein Gewächshaus mit einer Temperatur von 21 bis 23°C und einer relativen Luftfeuchtigkeit von ca. 70% gebracht.

10 Tage nach der Inokulation wird der Befall der Sämlinge bestimmt. Die erhaltenen Boniturwerte werden in Prozent Befall umgerechnet. 0% bedeutet keinen Befall, 100% bedeutet, daß die Pflanzen vollständig befallen sind.

Wirkstoffe, Wirkstoffkonzentrationen und Ergebnisse werden ermittelt. Bei diesem Test zeigen z.B. folgende Verbindungen eine sehr gute Wirkung, die derjenigen der aus dem Stand der Technik bekannten Verbindungen überlegen ist:

Verbindungen gemäß Herstellungsbeispielen: 4, 3, 5, 6, 7 und 8.

Herstellungsbeispiele

Beispiel 1

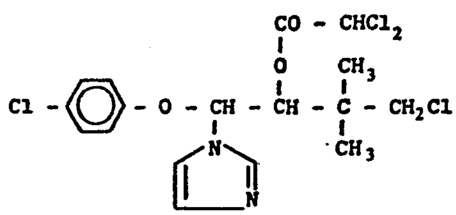

(Verfahren a)

19,8g (0,06 Mol) 1-(4-Chlorphenoxy)-1-(imidazol-1-yl)-3,3-dimethyl-4-chlorbutan-2-ol werden bei 0°C zu 200 ml Dichloracetylchlorid gegeben. Man läßt 24 Stunden bei Raumtemperatur rühren und destilliert des überschüssige Dichloracetylchlorid im Vakuum ab. Das zurückbleibende Oel wird in 400 ml Methylenchlorid aufgenommen, mit 500 ml wäßriger Natriumhydrogencarbonatlösung neutralisiert, die organische Phase abgetrennt, mit 200 ml Wasser gewaschen und über Natriumsulfat getrocknet. Das Lösungsmittel wird abdestilliert und der Rückstand in 200 ml Diisopropyläther aufgenommen. Nach mehrstündigem Stehen fallen farblose Kristalle aus. Man erhält 7,5g (29% der Theorie) 1-(4 Phenoxy) - 1 - (imidazol - 1 - yl) - 2 - dichloracetoxy - 3,3 - dimethyl - 4 - chlor - butan vom Schmelzpunkt 108—110°C.

*Herstellung der Vorstufen*

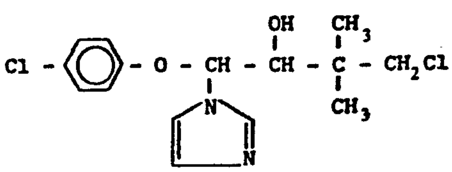

18,8 g (0,04 Mol) 1-(4-Chlorphenoxy)-1-(imidazol-1-yl)-3,3-dimethyl-4-chlor-butan-2-on-naphthalindisulfonat-(1,5) werden in 100 ml Methylenchlorid suspendiert und mit 100 ml Natriumhydrogencarbonatlösung versetzt. Die organische Phase wird abgetrennt, über Natriumsulfat getrocknet und durch Abdestillieren des Lösungsmittels im Vakuum eingeengt. Die so erhaltene Base wird in 100 ml Isopropanol aufgenommen und bei 5 bis 10°C werden 2 g (0,05 Mol) Natriumborhydrid portionsweise eingetragen. Man läßt 15 Stunden bei Raumtemperatur rühren und destilliert dann das Isopropanol ab. Der Rückstand wird in 100 ml Methylenchlorid aufgenommen und nach Zugabe von 100

ml Wasser weitere 15 Stunden bei Raumtemperatur gerührt. Anschließend wird die organische Phase abgetrennt, zweimal mit je 50 ml Wasser gewaschen, über Natriumsulfat getrocknet und eingeengt. Das zurückbleibende Oel wird in 100 ml Petroläther aufgekocht, wobei es zur Kristallisation kommt. Man erhält 9,8 g (75% der Theorie) 1-(4-Chlorphenoxy)-1-(imidazol-1-yl)-3,3-dimethyl-4-chlor-butan-2-ol vom Schmelzpunkt 120—125°C.

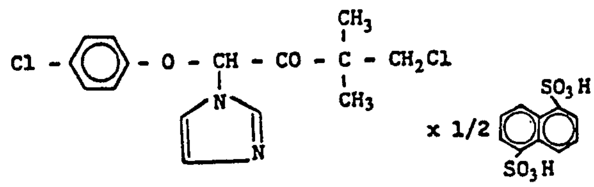

17 g (0,05 Mol) rohes 1-Brom-1-(4-chlorphenoxy)-3,3-dimethyl-4-chlor-butan-2-on werden in 100 ml absolutem Acetonitril gelöst. Dazu gibt man 12 g (0,175 Mol) Imidazol und erhitzt 40 Stunden unter Rückfluß. Danach wird durch Abdestillieren des Lösungsmittels im Vakuum eingeengt und der Rückstand in 300 ml Methylenchlorid aufgenommen. Man wäscht dreimal mit je 100 ml Wasser, trocknet über Natriumsulfat und engt erneut im Vakuum ein. Der Rückstand wird mit 100 ml Aceton aufenommen und mit einer Lösung von 9 g (0,038 Mol) 1,5-Naphthalindisulfonsäure in 50 ml Aceton versetzt. Nach 2 Stunden, wird der entstandene Niederschlag abgesaugt und getrocknet. Man erhält 19,8g (80,7% der Theorie) 1-(4-Chlorphenoxy)-1-(imidazol-1-yl)-3,3-dimethyl-4-chlor-butan-2-on-naphthalindisulfonat-(1,5) vom Schmelzpunkt 266—267°C.

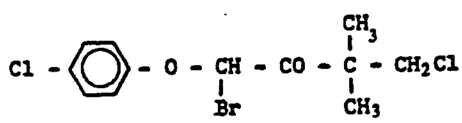

213,5 g (1 Mol) 1-Brom-4-chlor-3,3-dimethyl-butan-2-on werden zu einer siedenden Suspension von 128,5 g (1 Mol) 4-Chlorphenol und 140 g (1 Mol) Kaliumcarbonat in 1000 ml absolutem Aceton getropft. Man rührt 15 Stunden unter Rückfluß, läßt dann abkühlen, filtriert den anorganischen Rückstand ab und wäscht mit Aceton nach. Das Filtrat wird durch Abdestillieren des Lösungsmittels im Vakuum eingeengt und der Rückstand in 1000 ml Methylenchlorid aufgenommen, dreimal mit je 250 ml Wasser gewaschen, über Natriumsulfat getrocknet und destilliert. Man erhält 210 g (80,7% der Theorie) 4-Chlor-1-(4-chlorphenoxy)-3,3-dimethyl-butan-2-on vom Siedepunkt 125—127°C/0,1 mm.

210 g (0,81 Mol) 4-Chlor-1-(4-chlorphenoxy)-3,3-dimethyl-butan-2-on werden in 1000 ml Tetrachlorkohlenstoff gelöst. Bei Raumtemperatur werden 41 ml (0,01 Mol) Brom so zugetropft, daß stetiger Verbrauch eintritt. Anschließend läßt man 30 Minuten bei Raumtemperatur rühren. Nach Abdestillieren des Lösungsmittels im Vakuum erhält man 268,3 g (98% der Theorie) rohes 1-Brom-4-chlor-1-(4-chlorphenoxy)-3,3-dimethyl-butan-2-on, das direkt weiter umgesetzt wird.

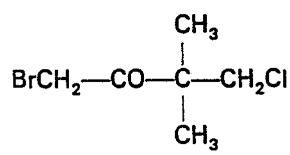

134,5 g (1 Mol) 1-Chlor-2,2-dimethyl-butan-3-on werden in 500 ml Aether gelöst. Bei Raumtemperatur werden unter leichter Kühlung 51 ml (1 Mol) Brom so zugetropft, daß stetiger Verbrauch eintritt. Danach wird die Lösung in 1000 ml Eiswasser eingerührt, die organische Phase abgetrennt, mit 250 ml Wasser nachgewaschen, über Natriumsulfat getrocknet und destilliert. Man erhält 169 g (80% der Theorie) 1 - Brom - 4 - chlor - 3,3 - dimethyl - butan - 2 - on vom Siedepunkt 95—106°C/13mm.

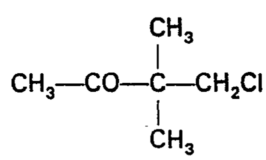

11,6 g (0,1 Mol) 2,2-Dimethyl-1-hydroxy-butan-3-on werden bei 50 bis 60°C (Eiskühlung) zu 20,5 g (0,1 Mol) N,N-Diäthyl-1,2,2-trichlorvinylamin getropft. Nach zweistündigem Rühren bei 60°C wird im Wasserstrahlvakuum destilliert. Man erhält 8,1 g (60% der Theorie) 1-Chlor-2,2-dimethylbutan-3-on vom Schmelzpunkt 60—62°C/12mm.

(Das 1-Chlor-2,2-dimethyl-butan-3-on wird in einer Ausbeute von 90% erhalten, wenn man äquimolare Mengen 2,2-Dimethyl-1-hydroxy-butan-3-on und Triphenylphosphin in der zehnfachen Menge Tetrachlorkohlenstoff 12 Stunden unter Rückfluß erhitzt, das Lösungsmittel abdestilliert, den Rückstand in Aether aufnimmt, filtriert und destilliert.)

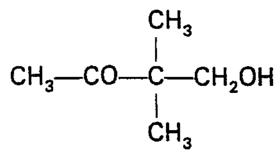

Zu 172 g (2 Mol) Methylisopropylketon in 1000 ml Methanol werden 66 g (2,2 Mol) Para-Formaldehyd und 1 g Kaliumhyroxid in 10 ml Methanol getropft. Man erhitzt 15 Stunden unter Rückfluß und destilliert anschließend das Methanol über eine Kolonne bei 82°C Innentemperatur ab. Der Rückstand wird im Wasserstrahlvakuum destilliert. Man erhält 152,7 g (68% der Theorie) 2,2-Dimethyl-1-hydroxy-butan-3-on vom Siedepunkt 80—82°C/12mm.

Beispiel 2

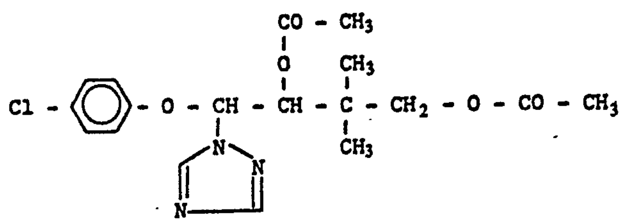

(Verfahren a)

46 g (0,123 Mol) 1-(4-Chlorphenoxy)-1-(1,2,4-triazol-1-yl)-3,3-dimethyl-butan-2,4-diol werden mit 16 g (0,205 Mol) Acetylchlorid ca.16 Stunden auf 50°C erwärmt. Danach werden 500 ml Methylenchlorid zugegeben und mit 1000 ml gesättigter Natriumhydrogencarbonatlösung versetzt. Man läßt 1 Stunde bei Raumtemperatur rühren, trennt die organische Phase ab, wäscht sie zweimal mit je 500 ml Wasser, trocknet über Natriumsulfat und engt durch Abdestillieren des Lösungsmittels im Vakuum ein. Das zurückbleibende Oel wird in 300 ml Aceton aufgenommen und mit 23g Naphthalindisulfonsäure-(1,5) versetzt. Der entstehende Niederschlag wird abgesaugt, in 600 ml Methylenchlorid aufgenommen und mit 1000 ml gesättigter Natriumhydrogencarbonatlösung neutralisiert. Man trennt die organische Phase ab, wäscht zweimal mit je 500 ml Wasser und trocknet über Natriumsulfat. Danach wird durch Abdestillieren des Lösungsmittels im Vakuum eingeengt. Das zurückbleibende Oel kristallisiert nach der Zugabe von 100 ml Diisopropyläther. Man erhält 14g (29% der Theorie) 1-(4-Chlorphenoxy)-1-(1,2,4-triazol-1-yl)-3,3-dimethyl-2,4-diacetoxybutan vom Schmelzpunkt 84—87°C.

*Herstellung der Vorstufen*

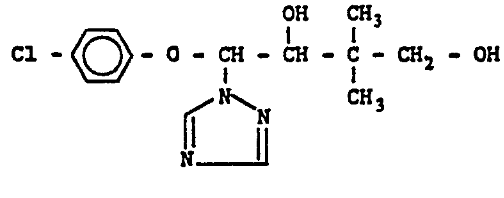

25 g (0,08 Mol) 1-(4-Chlorphenoxy)-1-(1,2,4-triazol-1-yl)-3,3-dimethyl-4-hydroxy-butan-2-on werden in 350 ml Isopropanol gelöst und 3,5 g Natriumborhydrid portionsweise bei Raumtemperatur zugegeben. Man läßt 15 Stunden bei Raumtemperatur rühren, gibt 500 ml Wasser zu, läßt weitere 15 Stunden bei Raumtemperatur rühren, gibt 300 ml Methylenchlorid hinzu und wäscht die organische Phase dreimal mit je 100 ml Wasser. Die organische Phase wird über Natriumsulfat getrocknet, im Wasserstrahlvakuum das Lösungsmittel abdestilliert und der Rückstand mit 100 ml Aether versetzt. Man erhält 17 g (67,6% der Theorie) 1-(4-Chlorphenoxy)-1-(1,2,4-triazol-1-yl)-3,3-dimethyl-butan-2,4-diol als farblose Kristalle vom Schmelzpunkt 110—112°C.

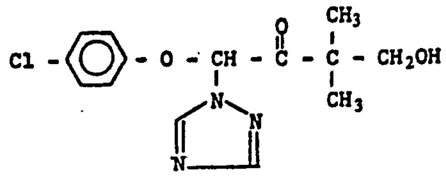

77,7 g (0,225 Mol) 1-(4-Chlorphenoxy)-1-(1,2,4-triazol-1-yl)-3,3-dimethyl-4-acetoxy-butan-2-on werden in 500ml Methanol gelöst, 27 ml konzentrierte Salzsäure zugegeben und 8 Stunden unter Rückfluß erhitzt. Man destilliert das Lösungsmittel im Wasserstrahlvakuum ab, nimmt in 500 ml Methylenchlorid auf, verrührt mit 500 ml wässriger, gesättigter Natriumhydrogencarbonatlösung, trennt die organische Phase ab, wäscht dreimal mit je 100 ml Wasser und destilliert das Lösungsmittel im Vakuum ab. Man versetzt den Rückstand mit 200 ml Petroläther, saugt die ausgefallenen Kristalle ab und trocknet bei 40°C im Umlufttrockenschrank.

Man erhält 60,8 g (87,5% der Theorie) 1-(4-Chlorphenoxy)-1-(1,2,4-triazol-1-yl)-3,3-dimethyl-4-hydroxy-butan-2-on vom Schmelzpunkt 110—111°C.

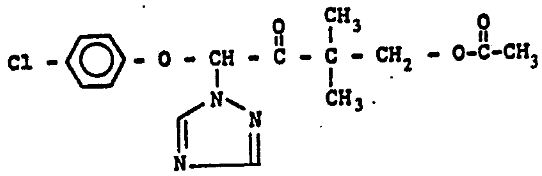

Die Ausgangsverbindung 1-(4-Chlorphenoxy)-1-(1,2,4-triazol-1-yl)-3,3-dimethyl-4-acetoxy-butan-2-on wird ausgehend von Methyl-isopropyl-keton durch Formylierung, Veresterung mit Essigsäureanhydrid, Bromierung, nucleophile Substitution mit p-Chlorphenol, Bromierung und nucleophile Substitution mit 1,2,4-Triazol erhalten (vgl. auch DE—OS 26 35 666).

Beispiel 3

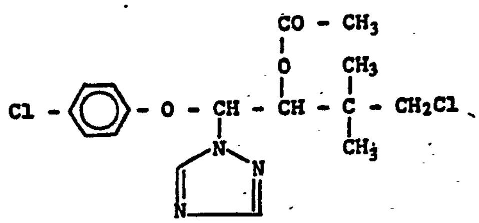

(Verfahren b)

23,1 g (0,07 Mol) 1-(4-Chlorphenoxy)-1-(1,2,4-triazol-1-yl)-3,3-dimethyl-4-chlor-butan-2-ol werden in 100 ml Essigsäureanhydrid gelöst und 12 Stunden auf 100°C erhitzt. Danach wird durch Abdestillieren des überschlüssigen Essigsäureanhydrids im Vakuum eingeengt. Das zurückbleibende Oel nimmt man in 150 ml Diisopropyläther auf und läßt es bei 0°C auskristallisieren. Man erhält 17 g (65% der Theorie) 1-(4-Chlorphenoxy)-1-(1,2,4-triazol-1-yl)-2-acetoxy-3,3-dimethyl-4-chlor-butan vom Schmelzpunkt 111—117°C.

Entsprechend werden die Verbindungen der folgenden Tabelle erhalten.

TABELLE

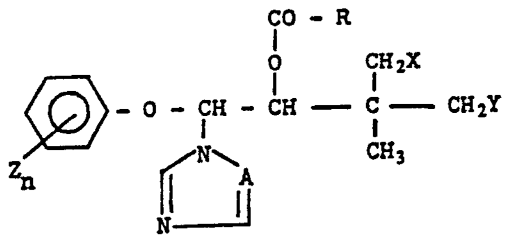

| Bsp. No. | A | R | X | Y | $Z_n$ | Schmelzpunkt (°C) |
|---|---|---|---|---|---|---|
| 4 | N | $-CHCl_2$ | H | Br | 4-Cl | 97–100 |
| 5 | N | $-CH_2Cl$ | H | Cl | 4-Cl | 123–125 |
| 6 | N | $-CHCl_2$ | H | Cl | 4-Cl | 74–80 |
| 7 | N | $CH_3$ | H | Br | 4-Cl | 101–106 |
| 8 | N | $-CH_2Cl$ | H | Br | 4-Cl | 120–122 |
| 9 | CH | $CH_3$ | H | Cl | 4-Cl | 121–123 |
| 10 | CH | $CH_3$ | H | Br | 4-Cl | 188–191 (×1/2 NDS) |
| 11 | N | $CH_3$ | H | Cl | 4 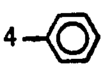 | 84–97 (B-Form) |
| 12 | N | 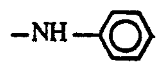 | H | Cl | 4 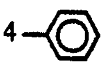 | 160–10 (Zers.) |
| 13 | N | 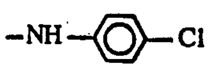 | H | Cl | 4 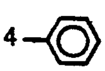 | 165–67 (A-Form) |
| 14 | N | $-NHCH_3$ | H | Cl | 4 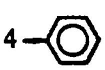 | 120–24 |
| 15 | N | $-NHCH_3$ | H | Br | 4 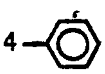 | 125–30 |
| 16 | N | $-NHCH_3$ | H | Cl | 4-Cl; 2-$CH_3$ | 135–37 |
| 17 | N | $-NHCH_3$ | H | Br | 2-Cl | 120–23 |
| 18 | N | $-NHCH_3$ | H | Br | 4-Cl, 2-$CH_3$ | 131–32 |

NDS = Naphthalindisulfonsäure-(1,5)
A– und B-Form: Jeweils eine der beiden möglichen geometrischen Isomeren

**Patentansprüche**

1. Acylierte 1-Azolyl-2-hydroxy-butan-Derivate der allgemeinen Formel I

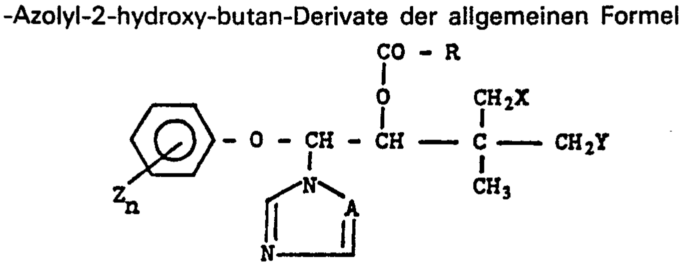

(I)

in welcher

A für ein Stickstoffatom oder die CH-Gruppe steht,

R für Alkyl mit 1 bis 6, Alkenyl und Alkinyl mit jeweils 2 bis 4 Kohlenstoffatomen, Halogenalkyl mit 1 bis 2 Kohlenstoff- und 1 bis 5 Halogenatomen, Alkoxy und Alkoxyalkyl mit 1 bis 4 Kohlenstoffatomen in den Alkylteilen steht, ferner für Phenyl und Phenylalkyl mit bis zu 2 Kohlenstoffatomen im Alkylteil steht, wobei die beiden letztgenannten Reste im Phenylteil substituiert sein können durch Halogen, Cyano, Nitro, Methyl und Ethyl, ferner für Alkylamino mit 1 bis 12 Kohlenstoffatomen und für Dimethyl- oder Diäthylamino, Halogenalkylamino mit bis zu 4 Kohlenstoff- und 5 Halogenatomen steht, für Alkoxycarbonylamino und Alkoxyalkylamino mit jeweils 1 bis 4 Kohlenstoffatomen in jedem Alkylteil und endlich für Phenylamino steht, welches gegebenenfalls durch Halogen, Nitro, Cyano, Alkyl mit bis zu 4, Alkoxy und Alkylthio mit bis zu 2 Kohlenstoffatomen, Halogenalkyl mit bis zu 2 Kohlenstoff- und 5 Halogenatomen und durch Alkoxycarbonylalkenyl mit bis zu 4 Kohlenstoffatomen in den Alkyl- und Alkenylteilen substituiert sein kann;

X und Y für Halogen und Alkylcarbonyloxy mit 1 bis 4 Kohlenstoffatomen im Alkylteil stehen und darüber hinaus X noch für Wasserstoff stehen kann,

Z für Halogen, Cyano, Nitro, Alkyl mit bis zu 4 Kohlenstoffatomen, für Cyclohexyl, für Halogenalkyl mit bis zu 2 Kohlenstoff- und 5 Halogenatomen, für Alkoxy mit bis zu 2 und Alkoxycarbonyl mit insgesamt bis zu 5 Kohlenstoffatomen steht, und weiterhin für Phenyl oder Phenoxy steht, wobei diese beiden Reste gegebenenfalls durch Halogen, Amino, Cyano, Nitro, Methyl und Ethyl substituiert sein können, und

n für Zahlen von 0 bis 3 steht,

und deren physiologisch verträgliche Säureadditions-Salze und Metallsalz-Komplexe.

2. Verfahren zur Herstellung von acylierten 1-Azolylhydroxy-butan-Derivaten, dadurch gekennzeichnet, daß man 1-Azolyl-2-hydroxy-butan-Derivate der allgemeinen Formel II

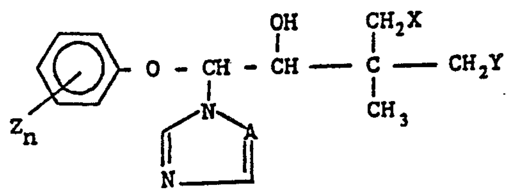

(II)

in welcher

A, X, Y, Z und n die in Anspruch 1 angegebene Bedeutung haben,

(a) mit Säurehalogeniden der allgemeinen Formel II

Hal—CO—R (III)

in welcher

R die in Anspruch 1 angegebene Bedeutung hat und Hal für Halogen steht, gegebenenfalls in Gegenwart eines Lösungsmittels und gegebenenfalls in Gegenwart eines Säurebindemittels umsetzt, oder

(b) mit Säureanhydriden der allgemeinen Formel IV

R—CO—O—CO—R (IV)

in welcher

R die in Anspruch 1 angegebene Bedeutung hat,

in Gegenwart eines Lösungsmittels und gegebenenfalls in Gegenwart eines Katalysators umsetzt, oder

(c) mit Ketenen der allgemeinen Formel V

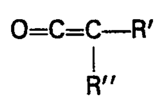

(V)

in welcher

R′ für Wasserstoff, für Alkyl mit bis zu 5 oder für Alkenyl oder Alkoxy mit bis zu 3 Kohlenstoffatomen steht, ferner für Phenyl steht, welches durch Halogen, Cyano, Nitro und Alkyl mit bis zu 2 Kohlenstoffatomen substituiert sein kann, sodann für Halogenmethyl mit 1 bis 3 Fluor- und/oder Chloratomen steht, und schließlich für Chlor oder Brom steht, und

R″ die unter R′ genannten Bedeutungen annehmen kann, jedoch mit der Maßgabe, daß die

Summe der Kohlenstoffatome in den Alkyl-, Alkenyl- und Alkoxy-Resten für R' und R'' gemeinsam um die Zahl 1 unter der Zahl derjenigen Kohlenstoffatome, die bei der R-Definition in Anspruch 1 für die genannten Reste angegeben ist, bleiben muß, und daß höchstens einer der Reste R' und R'' für gegebenenfalls wie angegeben substituiertes Phenyl oder Halogenmethyl stehen darf, in Gegenwart eines Lösungsmittels und gegebenenfalls in Gegenwart eines Katalysators umsetzt, oder

(d) mit Isocyanaten der allgemeinen Formel VI

$$O{=}C{=}N{-}R''' \quad (VI)$$

in welcher

R''' für Alkyl mit bis zu 6, für Halogenalkyl mit bis zu 2 Kohlenstoff- und bis zu 5 Halogenatomen steht, für Alkoxycarbonyl oder Alkoxyalkyl mit jeweils bis zu 4 Kohlenstoffatomen in jedem Alkylteil steht, oder für Phenyl steht, welches durch Halogen, Cyano, Nitro und Alkyl mit bis zu 4, Alkoxy und Alkylthio mit bis zu 2 Kohlenstoffatomen, Halogenalkyl mit bis zu 2 Kohlenstoff- und bis zu 5 Halogenatomen und/oder durch Alkoxycarbonylalkenyl mit bis zu 4 Kohlenstoffatomen im Alkyl- oder Alkenyl-Teil substituiert sein kann,

in Gegenwart eines Lösungsmittels und gegebenenfalls in Gegenwart eines Katalysators umsetzt.

3. Fungizide Mittel, gekennzeichnet durch einen Gehalt an mindestens einem acylierten Azolyl-2-hydroxy-butan-Derivat gemäß Anspruch 1.

4. Verwendung von acylierten Azolyl-2-hydroxy-butan-Derivaten gemäß Anspruch 1 zur Bekämpfung von Pilzen.

## Claims

1. Acylated 1-azolyl-2-hydroxy-butane derivatives of the general formula

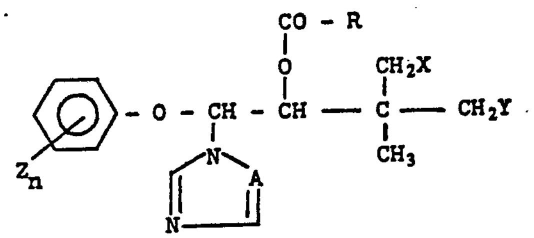

(I)

in which

A represents a nitrogen atom or the CH group,

R represents alkyl with 1 to 6, alkenyl and alkynyl with in each case 2 to 4 carbon atoms, halogenoalkyl with 1 to 2 carbon atoms and 1 to 5 halogen atoms, alkoxy and alkoxyalkyl with 1 to 4 carbon atoms in the alkyl parts, also represents phenyl and phenylalkyl with up to 2 carbon atoms in the alkyl part, it being possible for the two last-mentioned radicals to be substituted in the phenyl part by halogen, cyano, nitro, methyl and ethyl, also represents alkylamino with 1 to 12 carbon atoms and dimethylamino or diethylamino, halogenoalkylamino with up to 4 carbon atoms and 5 halogen atoms, represents alkoxycarbonylamino and alkoxyalkylamino with in each case 1 to 4 carbon atoms in each alkyl part and finally represents phenylamino, which can optionally be substituted by halogen, nitro, cyano, alkyl with up to 4, alkoxy and alkylthio with up to 2 carbon atoms, halogenoalkyl with up to 2 carbon atoms and 5 halogen atoms and by alkoxycarbonylalkenyl with up to 4 carbon atoms in the alkyl and alkenyl parts;

X and Y represent halogen and alkylcarbonyloxy with 1 to 4 carbon atoms in the alkyl part and furthermore X can also represent hydrogen,

Z represents halogen, cyano, nitro, alkyl with up to 4 carbon atoms, cyclohexyl, halogenoalkyl with up to 2 carbon atoms and 5 halogen atoms, alkoxy with up to 2 and alkoxycarbonyl with altogether up to 5 carbon atoms, and furthermore represents phenyl or phenoxy, it being possible for these two radicals optionally to also be substituted by halogen, amino, cyano, nitro, methyl and ethyl and

n represents numbers from 0 to 3,

and their physiologically acceptable acid addition salts and metal salt complexes.

2. Process for the preparation of acylated 1-azolyl-hydroxybutane derivatives, characterised in that 1-azolyl-2-hydroxybutane derivatives of the general formula II

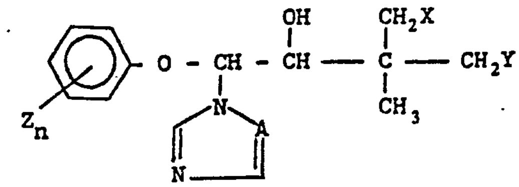 (II)

in which A, X, Y, Z and n have the meaning indicated in Claim 1,

(a) are reacted with acid halides of the general formula III

Hal—CO—R (III)

in which R has the meaning indicated in Claim 1 and Hal represents halogen, if appropriate in the presence of a solvent and if appropriate in the presence of an acid-binding agent, or

(b) are reacted with acid anhydrides of the general formula IV

R—CO—O—CO—R (IV)

in which R has the meaning indicated in Claim 1, in the presence of a solvent and if appropriate in the presence of a catalyst, or

(c) are reacted with ketenes of the general formula V

O=C=C—R'
 |
R'' (V)

in which R′ represents hydrogen, alkyl with up to 5 or alkenyl or alkoxy with up to 3 carbon atoms, further represents phenyl, which can be substituted by halogen, cyano, nitro and alkyl with up to 2 carbon atoms, further represents halogenomethyl with 1 to 3 fluorine and/or chlorine atoms, and finally represents chlorine or bromine, and

R″ can have the meanings mentioned under R′, however with the proviso that the sum of the carbon atoms in the alkyl, alkenyl and alkoxy radicals for R′ and R″ together has to remain lower than the number of those carbon atoms which is given for the named radicals in the definition of R in Claim 1, by the number 1, and that at most one of the radicals R′ and R″ may represent phenyl optionally substituted as indicated or halogenomethyl, in the presence of a solvent and if appropriate in the presence of a catalyst, or

(d) are reacted with isocyanates of the general formula IV

O=C=N—R‴ (VI)

in which R‴ represents alkyl with up to 6, halogenoalkyl with up to 2 carbon atoms and up to 5 halogen atoms, alkoxycarbonyl or alkoxyalkyl with in each case up to 4 carbon atoms in each alkyl part, or represents phenyl which can be substituted by halogen, cyano, nitro and alkyl with up to 4, alkoxy and alkylthio with up to 2 carbon atoms, halogenoalkyl with up to 2 carbon atoms and up to 5 halogen atoms and/or by alkoxycarbonylalkenyl with up to 4 carbon atoms in the alkyl or alkenyl part, in the presence of a solvent and if appropriate in the presence of a catalyst.

3. Fungicidal agents, characterised in that they contain at least one acylated azolyl-2-hydroxy-butane derivative according to Claim 1.

4. Use of acylated azolyl-2-hydroxy-butane derivatives according to Claim 1 for combating fungi.

**Revendications**

1. Dérivés acylés de 1-azolyl-2-hydroxy-butanes de formule générale:

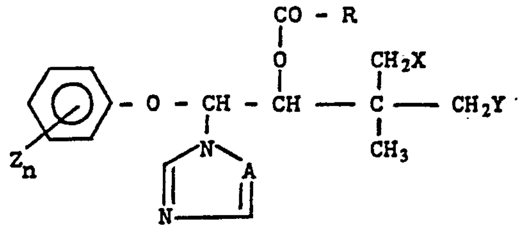

(I)

dans laquelle

A représente un atome d'azote ou le groupe CH, R est un groupe alkyle ayant 1 à 6 atomes de carbone, un groupe alcényle et un groupe alcynyle ayant chacun 2 à 4 atomes de carbone, un groupe halogénalkyle ayant 1 ou 2 atomes de carbone et 1 à 5 atomes d'halogènes, un groupe alkoxy et un groupe alkoxyalkyle ayant 1 à 4 atomes de carbone dans les parties alkyliques, en outre le groupe phényle et un groupe phénylalkyle ayant jusqu'à 2 atomess de carbone dans la partie alkyle, les deux restes mentionnés en dernier lieu pouvant être substitués dans la partie phényle par un halogène, un radical cyano, nitro, méthyle et éthyle, en outre un groupe alkylamino ayant 1 à 12 atomes de carbone et le groupe diméthyl- ou diéthylamino, un groupe halogénalkylamino ayant jusqu'à 4 atomes de carbone et jusq'à 5 atomes d'halogènes, un groupe alkoxycarbonylamino et alkoxyalkylamino ayant chacun 1 à 4 atomes des carbone dans chaque partie alkyle et enfin un groupe phénylamino qui peut être substitué le cas échéant par un halogène, un radical nitro, cyano, alkyle ayant jusqu'à 4, alkoxy et alkylthio ayant jusqu'à 2 atomes de carbone, un radical halogénalkyle ayant 2 atomes de carbone et jusqu'à 5 atomes d'halogènes et par un radical alkoxycarbonylalcényle ayant jusqu'à 4 atomes de carbone dans les parties alkylique et alcénylique; X et Y représentent un halogène et un groupe alkylcarbonyloxy ayant 1 à 4 atomes de carbone dans la partie alkylique et X pouvant encore représenter en outre de l'hydrogène, Z est un halogène, un groupe cyano, nitro, alkyle ayant jusqu'à 4 atomes de carbone, le groupe cyclohexyle, un groupe halogénalkyle ayant jusqu'à 2 atomes de carbone et 5 atomes d'halogènes, un groupe alkoxy ayant jusqu'à 2 et un groupe alkylcarbonyle ayant au total jusqu'à 5 atomes de carbone, et en outre le groupe phényle ou le groupe phénoxy, ces deux restes pouvant éventuellement être substitués par un halogène, un radical amino, cyano, nitro, méthyle et éthyle et

*n* représente les nombres de 0 à 3,

et leurs sels d'addition d'acides et leurs complexes de sels métalliques acceptables du point de vue physiologique.

2. Procédé de production de dérivés acylés de 1-azolylhydroxy-butanes, caractérisé en ce qu'on fait réagir des dérivés de 1-azolyl-2-hydroxy-butanes de formule générale II:

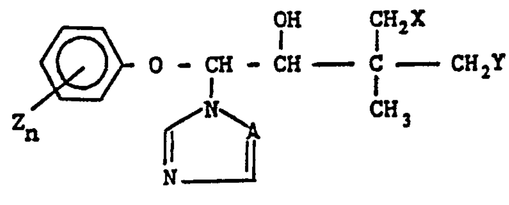

(II)

dans laquelle

A, X, Y, Z et *n* ont la définition indiquée dans la revendication 1,

(a) avec des halogénures d'acides de formule générale III:

$$Hal—CO—R \qquad (III)$$

dans laquelle

R a la définition indiquées dans la revendication 1 et Hal est un halogène,

éventuellement en présence d'un solvant et le cas échéant, en présence d'un accepteur d'acide ou

(b) avec des anhydrides d'acides de formule générale IV:

$$R—CO—O—CO—R \qquad (IV)$$

dans laquelle

R a la définition donnée dans la revendication 1, en présence d'un solvant et le cas échéant, en

présence d'un catalyseur, ou

(c) avec des cétènes de formule générale V:

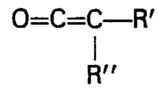

(V)

dans laquelle

R′ est l'hydrogène, un reste alkyle ayant jusqu'à 5 ou un reste alcényle ou alkoxy ayant jusqu'à 3 atomes de carbone, en outre le groupe phényle qui peut être substitué par un halogène, un radical cyano, nitro et alkyle ayant jusqu'à 2 atomes de carbone, en outre un reste halogénométhyle portant 1 à 3 atomes de fluor et/ou de chlore, et enfin du chlore ou du brome, et

R″ peut recevoir les définitions données pour R′, mais à condition que la somme des atomes de carbone des restes alkyle, alcényle et alkoxy pour R′ et R″ ensemble reste inférieure d'une unité au nombre d'atomes de carbone qui est indiqué pour les restes en question dans la définition de R dans la revendication 1, et qu'au maximum l'un des restes R′ et R″ représente un groupe phényle ou halogénométhyle éventuellement substitué comme indiqué, en présence d'un solvant et le cas échéant en présence d'un catalyseur, ou

(d) avec des isocyanates de formule générale VI:

$$O{=}C{=}N{-}R'''$$ (VI)

dans laquelle

R‴ est un reste alkyle ayant jusqu'à 6, un reste halogénalkyle ayant jusqu'à 2 atomes de carbone et jusqu'à 5 atomes d'halogènes, un reste alkoxycarbonyle ou alkoxyalkyle ayant chacun jusqu'à 4 atomes de carbone dans chaque partie alkyle, ou un reste phényle qui peut être substitué par un halogène, un radical cyano, nitro et alkyle ayant jusqu'à 4, alkoxy et alkylthio ayant jusqu'à 2 atomes de carbone, un reste halogénalkyle ayant jusqu'à 2 atomes de carbone et jusqu'à 5 atomes d'halogènes et/ou par un reste alkoxycarbonylalcényle ayant jusqu'à 4 atomes de carbone dans la partie alkyle ou alcényle,

en présence d'un solvant et le cas échéant, en présence d'un catalyseur.

3. Compositions fongicides, caractérisées par une teneur en au moins un dérivé acylé d'azolyl-2-hydroxy-butane suivant la revendication 1.

4. Utilisation de dérivés acylés d'azolyl-2-hydroxy-butanes suivant la revendication 1 pour la lutte contre des champignons.